# FASCICULE DE BREVET EUROPEEN

(11) **EP 1 252 900 B1**
(45) Date de publication et mention de la délivrance du brevet: **12.05.2004**
(21) Numéro de dépôt: 02362008.1
(22) Date de dépôt: 23.04.2002
(51) Int. Cl.: A61L 9/12, A61L 9/14

(54) **Dispositif de diffusion d'un produit volatil, notamment un parfum dans un espace de grand volume**
Vorrichtung zum Verdunsten eines flüchtigen Produkts, insbesondere eines Parfüms in Räumen mit grossen Volumen
Device for diffusing a volatile product, particularly a perfume in a large space

(30) Priorité: 23.04.2001 FR 0105450
(43) Date de publication de la demande: 30.10.2002
(73) Titulaire: Lenfant, Jean-Pierre, F-87600 Rochechouart (FR); Hiblot, Norbert, 56610 Arradon (FR)
(72) Inventeur: Lenfant, Jean-Pierre, 87600 Rochechouart (FR)
(74) Mandataire: Thébault, Jean-Louis

(56) Documents cités:
- DE-A- 4 409 598
- DE-U- 29 604 240
- FR-A- 2 797 189
- US-A- 5 302 359

## Description

La présente invention concerne un dispositif de diffusion d'un produit volatil, notamment un parfum dans un espace de grand volume tel qu'une pièce. De tels dispositifs sont connus par US-A-5 302 359, DE 29 604 240 U1, DE-A-4 409 598 et FR-A-2 797 189.

De plus en plus, les entreprises souhaitent personnaliser leurs espaces de vie et d'accueil en sorte de se forger une image de marque. C'est ainsi que des logos, des musiques, ou des décors spécifiques et propres à l'entreprise sont disposés dans ces lieux.

Un outil de personnalisation peut aussi être basé sur le sens olfactif et il est possible de concevoir un parfum dédié à une entreprise et conforme à son image et/ou aux produits qu'elle commercialise.

Ainsi, pour des établissements bancaires par exemple, il est possible de créer un parfum qui sera diffusé dans toutes les agences, si bien que les clients retrouveront dans chaque agence, le même environnement d'accueil, même sur le plan olfactif. Ceci est une signature pour le client qui assimile ce parfum à l'établissement bancaire qu'il a retenu.

Cette signature est également un élément de plaisir surtout en ville où les odeurs ne sont toujours des plus agréables.

Le problème reste de diffuser un tel parfum dans des conditions adaptées. Or ces conditions varient beaucoup d'un lieu à l'autre tant à cause du volume de la pièce, des effets de circulation d'air, du type de chauffage ou d'autres paramètres relatifs à la géométrie du lieu par exemple.

On sait que la diffusion statique de parfum est très irrégulière à cause des conditions environnementales comme les courants d'air, la température, le degré hygrométrique.

Il existe des systèmes à mèche qui peuvent pallier quelques irrégularités mais le résultat n'est pas satisfaisant.

De plus, quant on parle ambiance parfumée, il s'agit souvent de parfums dilués qui, par leur moindre puissance, contribuent à réduire partiellement les disparités de diffusion, mais ont pour inconvénient de ne pas restituer fidèlement le spectre olfactif.

De ce fait, la diffusion de parfum est très délicate par la concentration et la puissance dégagée.

Il faut en effet pouvoir distribuer des quantités très faibles de produit mais il faut parallèlement pouvoir les diluer dans des volumes d'air très importants, de façon homogène, pour obtenir la densité olfactive voulue.

Les parfums posent aussi un autre problème très délicat lors de la diffusion. En effet, la composition d'un parfum est telle qu'il comprend des substances de différentes volatilités. De ce fait, lors de la diffusion, les produits les plus légers vont s'évaporer plus rapidement modifiant au cours du temps le spectre olfactif. Il faut donc diffuser de très petites quantités mais dans leur intégralité.

De plus, il peut arriver que des produits lourds, entrant dans la composition des parfums ne s'évaporent pas ou très difficilement.

Enfin, il est nécessaire de prendre en compte les capacités corrosives très importantes des parfums vis à vis de nombreux matériaux.

Le dispositif selon la présente invention est particulièrement adapté pour pallier ces problèmes et pour permettre une diffusion d'un parfum dans un lieu de grand volume tel qu'un lieu public. Ce dispositif permet une diffusion homogène, une distribution de très faibles quantités et présente une grande fiabilité par la présence d'un nombre réduit de pièces en mouvement. Ce dispositif permet en outre de changer de parfum aisément sans qu'il y ait risque de mélange avec le parfum précédent.

Le présent dispositif est maintenant décrit en détail selon un mode de réalisation particulier, non limitatif, en regard des dessins annexés sur lesquels les différentes figures montrent :
- figure 1 : une vue d'un mode de réalisation particulier, et
- figures 2A et 2B : une variante d'agencement.

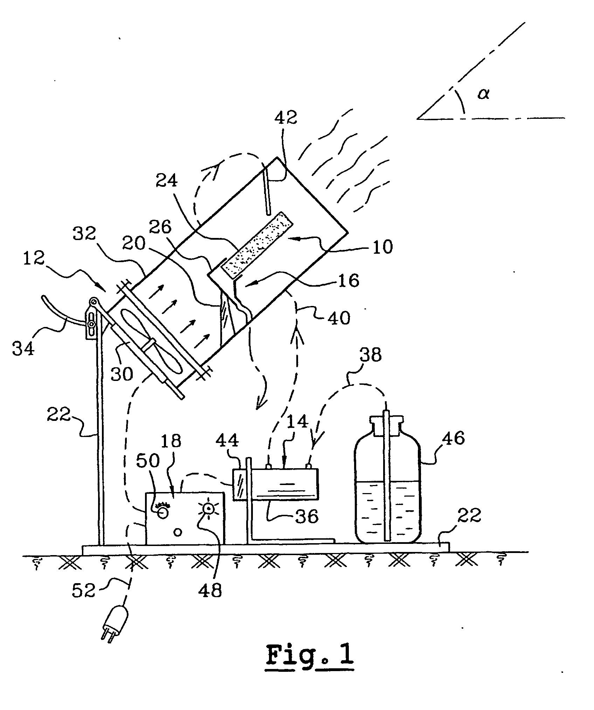

Le dispositif de diffusion de produits volatils, plus spécifiquement de parfum montré sur la figure 1, comprend des moyens 10 de répartition du produit, des moyens 12 de propagation forcée de ce produit, des moyens 14 de dosage du produit, des moyens 16 de récupération des produits non volatils et des moyens 18 de pilotage.

Les moyens 10 de répartition comprennent un support 20, solidaire indirectement d'un bâti 22. Ce support reçoit un élément 24 absorbant tel qu'un feutre ou une plaque céramique par exemple. Cet élément doit présenter des capacités de répartition très rapide du produit à diffuser. Il est disposé en un endroit donné par rapport aux moyens 12 de propagation forcée.

Dans le mode de réalisation préférentiel qui est représenté, l'optimisation et la recherche de compacité ont conduit à utiliser le support 20 pour y associer une gouttière 26 qui constitue les moyens 16 de récupération des produits non volatils comme cela sera expliqué plus avant.

Cette gouttière est reliée par une évacuation 28 comprenant un conduit et un contenant, non représenté, en sorte de collecter les écoulements.

Les moyens 12 de propagation forcée comprennent un ventilateur 30 disposé au fond d'un carter 32, lui-même solidarisé au bâti 22. Ce carter 32 est de section circulaire dans le mode de réalisation représenté et il est incliné suivant un angle par rapport à l'horizontal. Cette inclinaison moyennant l'interposition d'une articulation 34 à blocage peut être ajustée à l'azimut recherché.

Ce carter 32 reçoit le support 20 des moyens 10 de répartition. Ce support est placé dans ce carter, sensiblement dans la veine centrale, en offrant une surface de diffusion adaptée tout en perturbant le moins possible la propagation. Le support est tel que l'élément absorbant se trouve également incliné du même angle α afin que le produit à diffuser subisse les effets de la gravité.

Les moyens 14 de dosage du produit comprennent un système 36 de pompage/dosage, une alimentation 38 et une sortie 40 qui se termine par une canule 42 de distribution.

Cette canule, de petit diamètre, est solidaire du carter 32 qu'elle traverse pour que son extrémité vienne au droit de l'élément 24 d'absorption, de préférence, en partie haute.

Le système 36 de pompage/dosage est particulier car il doit répondre à des impératifs spécifiques. En effet, compte tenu des très petits volumes à délivrer, par exemple 0,1 mm³/mn, et avec une distribution échelonnée, une pompe à actionnement avec un tiroir de distribution et un électro-aimant 44 de manoeuvre est adaptée. Il faut tenir compte des risques de désamorçage et du fait que dans le cas où le produit est un parfum, la corrosion est très active.

Une réserve 46 permet d'alimenter la pompe.

Selon un mode de réalisation préféré, le système de pompage/dosage est une pompe de type péristatique. A cet effet, une partie du conduit allant de la réserve 46 à la canule 42 est souple et le système 36 comprend deux galets presseurs susceptibles de manière alternée de presser le conduit souple et d'entraîner le liquide vers la canule. De préférence, les galets sont montés de manière excentrée par rapport à un axe de rotation et par rotation sont susceptibles de presser le conduit jusqu'à obturation, le mouvement de rotation et de pressage simultané provoquant un déplacement du fluide présent dans le conduit. Cet agencement permet sans l'utilisation d'une vanne d'isoler le fluide situé en amont du fluide situé en aval. Ainsi, il permet de réduire le nombre de pièces en contact avec le fluide au seul conduit, ce qui limite les risques d'encrassement et de corrosion.

Quant aux moyens 18 de pilotage, ils comprennent des moyens 48 et 50 de réglage qui permettent d'ajuster le fonctionnement des différents moyens actifs, c'est-à-dire l'espace entre les impulsions des moyens de pompage/dosage donc le volume de produit distribué et la vitesse du ventilateur, c'est-à-dire le volume d'air diffusé. La combinaison de ces deux paramètres donne le pouvoir diffusant.

Ces moyens de pilotage sont alimentés par le réseau électrique général en 52.

Le fonctionnement de ce dispositif est maintenant décrit en détail, relativement à une diffusion d'un parfum dans un lieu public tel que la salle des guichets d'une banque.

L'utilisateur choisit un parfum parmi des parfums d'ambiance proposés ou fait élaborer un parfum à son image.

Ce parfum présente des caractéristiques spécifiques de volatilité et de pouvoir odoriférant.

Le parfum est livré dans un contenant adapté et étanche bien entendu afin de ne pas provoquer d'évaporation parasite.

Un élément 24 absorbant, neuf, est mis en place dans le support 26.

Le ventilateur est mis en marche et sa vitesse donc le débit d'air est réglé sur une valeur donné assez faible.

Le système 36 de pompage/dosage est alimenté en énergie électrique, ce qui conduit à la distribution de gouttes de parfum sur l'élément 24 absorbant.

Le parfum qui est distribué sur cet élément 24 absorbant et incliné est soumis à l'action du flux d'air du ventilateur, ce qui produit une évaporation d'une partie de ce parfum correspondant aux produits volatils que l'on souhaite diffuser.

Ce parfum migre sur tout l'élément et se répartit sur une grande surface, ce qui assure une meilleure diffusion, plus homogène. Ceci évite une trop forte ségrégation et permet d'obtenir les réelles senteurs du parfum tel qu'il a été conçu, avec la majorité de ses composés, les plus volatils sous les effets de la diffusion forcée, entraînant les moins volatils.

Le fait d'avoir un élément 24 incliné et une distribution de parfum via la canule 42 en partie haute dudit élément 24 permet de générer au niveau de l'élément 24 absorbant un flux de propagation en sens contraire par rapport au flux d'air généré par le ventilateur, ce qui contribue à améliorer l'échange et l'évaporation du parfum.

Par contre, une fraction très faible reste néanmoins non diffusée, fraction qu'il faut évacuer.

Comme l'élément 24 est incliné, ces produits non volatils comme des huiles, ont tendance à s'accumuler sous les effets de la gravité au bord inférieur de cet élément.

Lorsque les forces de capillarité ne sont plus suffisantes, les gouttes de ces produits non volatils tombent dans la gouttière 26 qui les collecte et les conduit vers un récipient adapté, non représenté.

En fonction du lieu, l'azimut du carter 32 est ajusté afin de permettre une diffusion parfaitement adaptée.

De plus, si nécessaire, pour obtenir une homogénéité plus grande encore, il est possible de monter le carter sur un bâti équipé de moyens de mise en rotation alternative, dans le plan horizontal pour couvrir tout le volume de la pièce.

Enfin, une fois en place et des essais effectués, on ajuste finement le volume de parfum distribué et/ou le volume d'air forcé diffusé.

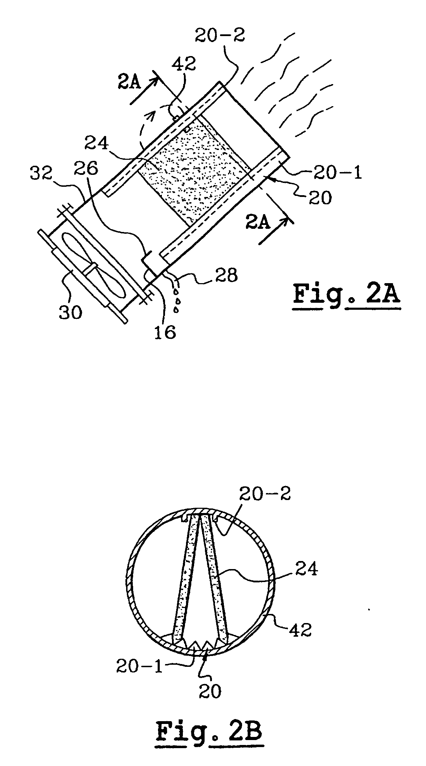

Sur les figures 2A et 2B, on a représenté une variante de réalisation qui présente plusieurs perfectionnements.

Ainsi dans le carter 32, il est prévu un support 20 en deux parties. La première partie comprend un profilé 20-2, par exemple en U renversé, et la seconde partie comprend un profilé 20-1 à au moins deux ondulations qui suit le profil du carter 32.

Ces deux profilés sont centrés sur un même diamètre, orienté verticalement pour bénéficier des effets de la gravité.

Cet agencement comprend également une gouttière 26 qui constitue les moyens 16 de récupération des produits non volatils, mais cette gouttière est déplacée pour être positionnée en aval du profilé 20-1 à au moins deux ondulations en sorte de recueillir les écoulements de produits non ou peu volatils.

Cette gouttière est reliée comme précédemment à une évacuation 28 comprenant un conduit et un contenant, non représenté, en sorte de collecter ces écoulements.

Ainsi que cela est visible sur la coupe transversale de la figure 2B, on peut disposer plusieurs éléments 24 absorbants dans ce mode de réalisation. Il n'y a aucun risque de gouttes éventuellement détachées des éléments absorbants au cours du fonctionnement, même en cas d'incident de fonctionnement ou de saturation passagère pour quelque raison que ce soit, les profilés 20-1 à ondulations formant un collecteur.

Le changement des éléments absorbants reste toujours très aisé par la face avant ouverte du carter 32.

On note aussi que les éléments 24 absorbants ne perturbent pas l'écoulement des flux d'air par les supports qui se trouvent dans cette variante en paroi et non dans le flux central, d'où l'obtention de flux plus laminaires qui assurent encore une meilleure diffusion.

En variante, on peut prévoir un ou plusieurs éléments absorbants plissés, de préférence avec des plis parallèles au flux d'air de manière à augmenter la surface d'échange sans toutefois perturber le flux d'air.

Il est à noter que la nature de l'élément absorbant permet d'obtenir des résultats plus ou moins satisfaisants en fonction des parfums. Néanmoins, des tests ont montré que l'effet de rétention doit être ajusté. Un essai montre qu'un matériau satisfaisant doit s'imprégner totalement du parfum au trempé et perdre sensiblement 1/3 du poids absorbé lorsqu'il est sorti du bain et mis en suspension.

L'ensemble du dispositif a été décrit de façon schématique mais il peut être regroupé dans un module de présentation adapté, avec une ergonomie et des formes assurant une parfaite intégration dans les lieux. Cet ensemble peut même être inséré dans du mobilier existant ou dans des créations architecturales spécifiques, constituant également un signe distinctif de l'exploitant.

Il est à noter que seuls le contenant 46 avec son nouveau parfum et les éléments 24 d'absorption doivent être changés, ce qui est très limité en coût mais aussi en temps.

## Revendications

1. Dispositif de diffusion d'une substance volatile comprise dans un produit tel qu'un parfum, ledit produit comprenant des substances de différentes volatilités, le dispositif comprenant des moyens (10) de répartition de ce produit, des moyens (12) de propagation forcée d'air le long des moyens de répartition et des moyens (14) de dosage de ce produit sur les moyens de répartition, **caractérisé en ce qu'**il comprend en outre des moyens (16) de récupération des substances non volatiles s'échappant des moyens de répartition.

2. Dispositif de diffusion selon la revendication 1, **caractérisé en ce qu'**il comprend des moyens (18) de pilotage des moyens (12) de propagation forcée et des moyens (14) de dosage.

3. Dispositif de diffusion selon l'une quelconque des revendications précédentes, **caractérisé en ce que** les moyens (10) de répartition comprennent un élément (24) absorbant, disposé dans un support (20).

4. Dispositif de diffusion selon la revendication 3, **caractérisé en ce que** l'élément absorbant (24) est incliné.

5. Dispositif de diffusion selon l'une quelconque des revendications précédentes, **caractérisé en ce que** les moyens (12) de propagation forcée d'air comprennent un carter (32) et un ventilateur (30) au fond de ce carter.

6. Dispositif de diffusion selon la revendication 5, **caractérisé en ce que** le carter (32) est monté sur un bâti (22) par une articulation (34) à blocage en sorte de régler l'inclinaison de ce carter par rapport à l'horizontale.

7. Dispositif de diffusion selon l'une quelconque des revendications précédentes, **caractérisé en ce que** les moyens (14) de dosage comprennent un système (36) de pompage/dosage, une alimentation (38) à partir d'un contenant (46) du produit à diffuser et une sortie (40) dont l'extrémité porte une canule (42) de distribution, à travers le carter (32), sur les moyens (10) de répartition.

8. Dispositif de diffusion selon les revendications 1, 5 et 7, **caractérisé en ce que** les moyens (18) de pilotage comprennent des moyens (48) et (50) de réglage pour ajuster le fonctionnement des moyens (36) de pompage/dosage et le ventilateur (30).

9. Dispositif de diffusion selon l'une quelconque des revendications précédentes, **caractérisé en ce que** les moyens (16) de récupération des substances non volatiles comprennent une gouttière (26) et une évacuation (28).

10. Dispositif de diffusion selon l'une quelconque des revendications précédentes, **caractérisé en ce que** les moyens (16) de récupération des substances non volatiles comprennent un support (20) en deux parties, un profilé (20-2) et un profilé (20-1) comprenant au moins deux ondulations, les deux profilés étant disposés centrés suivant un même diamètre, vertical par rapport au carter (32).

## Patentansprüche

1. Vorrichtung zum Verteilen einer flüchtigen Substanz, die in einem Produkt wie etwa einem Parfüm enthalten ist, wobei das Produkt Substanzen mit unterschiedlichem Flüchtigkeitsverhalten enthält, wobei die Vorrichtung Mittel (10) zum Zuteilen dieses Produkts, Mittel (12) zur erzwungenen Ausbreitung von Luft längs der Zuteilungsmittel und Mittel (14) zum Dosieren dieses Produkts in den Zuteilungsmitteln umfasst, **dadurch gekennzeichnet, dass** sie außerdem Mittel (16) umfasst, die nichtflüchtige Substanzen, die aus den Zuteilungsmitteln entweichen, wiedergewinnen.

2. Verteilungsvorrichtung nach Anspruch 1, **dadurch gekennzeichnet, dass** sie Mittel (18) zum Steuern der Mittel (12) zur erzwungenen Ausbreitung und der Dosierungsmittel (14) umfasst.

3. Verteilungsvorrichtung nach einem der vorhergehenden Ansprüche, **dadurch gekennzeichnet, dass** die Zuteilungsmittel (10) ein in einem Träger (20) angeordnetes absorbierendes Element (24) umfassen.

4. Verteilungsvorrichtung nach Anspruch 3, **dadurch gekennzeichnet, dass** das absorbierende Element (24) geneigt ist.

5. Verteilungsvorrichtung nach einem der vorhergehenden Ansprüche, **dadurch gekennzeichnet, dass** die Mittel (12) zur erzwungenen Luftausbreitung ein Gehäuse (32) und einen Ventilator (30) auf dem Boden dieses Gehäuses umfassen.

6. Verteilungsvorrichtung nach Anspruch 5, **dadurch gekennzeichnet, dass** das Gehäuse (32) durch ein Gelenk (34) mit Sperre an einem Rahmen (22) angebracht ist, derart, dass die Neigung dieses Gehäuses in Bezug auf die Horizontale eingestellt werden kann.

7. Verteilungsvorrichtung nach einem der vorhergehenden Ansprüche, **dadurch gekennzeichnet, dass** die Dosierungsmittel (14) ein Pump/Dosierungssystem (36), eine von einem Behältnis (46) für das zu verteilende Produkt ausgehende Versorgung (38) und einen Ausgang (40), dessen Ende in den Zuteilungsmitteln (10) eine durch das Gehäuse (32) verlaufende Verteilungskanüle (42) trägt, umfassen.

8. Verteilungsvorrichtung nach den Ansprüchen 1, 5 und 7, **dadurch gekennzeichnet, dass** die Steuermittel (18) Einstellmittel (48) und (50) zum Einstellen des Betriebs der Pump-/Dosierungsmittel (36) und des Ventilators (30) umfassen.

9. Verteilungsvorrichtung nach einem der vorhergehenden Ansprüche, **dadurch gekennzeichnet, dass** die Mittel (16) zur Wiedergewinnung nichtflüchtiger Substanzen eine Tropfrinne (26) und eine Entleerungseinrichtung (28) umfassen.

10. Verteilungsvorrichtung nach einem der vorhergehenden Ansprüche, **dadurch gekennzeichnet, dass** die Mittel (16) zur Wiedergewinnung nichtflüchtiger Substanzen einen zweiteiligen Träger (20), d. h. ein Profilelement (20-2) und ein Profilelement (20-1) umfassen, die ihrerseits wenigstens zwei gewellte Abschnitte umfassen und zentriert auf denselben Durchmesser, der in Bezug auf das Gehäuse (32) vertikal ist, angeordnet sind.

## Claims

1. A device for diffusing a volatile substance included in a product such as a perfume, said product comprising substances of various volatilities, the device comprising means (10) for distributing this product, means (12) for forced propagation of air along distribution means and means (14) for apportioning this product onto the distribution means, **characterised in that** it also comprises means (16) for recovering non-volatile substances escaping from the distribution means.

2. A diffusion device according to claim 1, **characterised in that** it comprises means (18) for controlling the forced propagation means (12) and the apportioning means (14).

3. A diffusion device according to any one of the preceding claims, **characterised in that** the distribution means (10) comprises an absorbent element (24), disposed in a support (20).

4. A diffusion device according to claim 3, **characterised in that** the absorbent element (24) is inclined.

5. A diffusion device according to any one of the preceding claims, **characterised in that** the forced air propagation means (12) comprises a casing (32) and a fan (30) at the bottom of this casing.

6. A diffusion device according to claim 5, **characterised in that** the casing (32) is mounted on a frame (22) by means of an articulation (34) with locking so as to adjust the inclination of this casing with respect to the horizontal.

7. A diffusion device according to any one of the preceding claims, **characterised in that** the apportioning means (14) comprises a pumping/apportioning system (36), a supply (38) from a container (46) of the product to be diffused and an outlet (40) whose end carries a cannula (42) for distribution, through the casing (32), on the distribution means (10).

8. A diffusion device according to claims 1, 5 and 7, **characterised in that** the control means (18) comprise adjustment means (48) and (50) for adjusting the functioning of the pumping/apportioning means (36) and the fan (30).

9. A diffusion device according to any one of the preceding claims, **characterised in that** the means (16) for recovering non-volatile substances comprise a channel (26) and a discharge (28).

10. A diffusion device according to any one of the preceding claims, **characterised in that** the means (16) for recovering non-volatile substances comprises a support (20) in two parts, a profile (20-2) and a profile (20-1) comprising at least two corrugations, the two profiles being disposed centred on the same diameter, vertical with respect to the casing (32).
